# EUROPEAN PATENT APPLICATION

(11) **EP 4 344 655 A1**
(43) Date of publication of application: **03.04.2024**
(21) Application number: 22275128.1
(22) Date of filing: 27.09.2022
(51) Int. Cl.: A61B 17/12, A61M 25/00, A61F 2/958, A61M 25/06

(54) **CATHETER DEVICE WITH IMPROVED SHEATH DESIGN**

(71) Applicant: Hilmy, Shereef, Mission, TX 78572 (US)
(72) Inventor: Hilmy, Shereef, Mission, TX 78572 (US)
(74) Representative: DREISS Patentanwälte PartG mbB

(57) **Abstract**

A vascular catheter assembly for percutaneous carotid artery stent placement through trans-femoral access is provided in combination with an introducer dilator device. The catheter assembly includes a catheter sheath having a sidewall construction formed as a multi-layered laminate arrangement. A dual-port cross-cut valve is mounted to the proximal end of the catheter sheath. A first valve port communicates with a micro-tube passageway that is formed in the sidewall construction and is adapted to access an occlusion balloon carried at a distal end of the catheter sheath. Fluid supplied through the first valve port is transported via the passageway to the balloon to inflate it. A second valve port communicates with the catheter sheath lumen, allowing blood products to backflow through the catheter sheath lumen and exit the valve to a filter system. The dilator accesses the catheter sheath lumen via the valve.

## Description

### Field of the Invention

The present invention relates generally to medical instruments, and more particularly, to a catheter assembly having a sheath design incorporating various features to facilitate a range of interventional procedures such as trans-femoral carotid artery stent placement.

### Background of the Invention,

For many individuals, maintaining a healthy lifestyle is critical to their longevity. While some are born more susceptible to disease than others, it is scientifically proven that practicing healthy habits will benefit everyone. According to Monique Tello's Harvard Health Publishing article, five areas that have been researched to decrease the risk of premature death or illness and are thus considered building blocks of a healthy lifestyle include: a healthy diet, healthy physical activity level, maintaining a healthy body weight, never smoking, and keeping alcohol intake to a minimum. A healthy diet consists of well-balanced meals, including servings of vegetables, fruits, whole grains, healthy fats, protein, and other vitamin-rich foods, while avoiding excessive amounts of sugar, trans fat, and processed meats. As for physical activity level, moderate exercise for 150 minutes per week is recommended by the U.S. Department of Health and Human Services.

As attainable as these goals may sound, the majority of Americans do not successfully follow these guidelines. For example, the Department of Health and Human Services found that over 80% of adults do not meet the recommendation for both aerobic and muscle strengthening activities, while adolescents to not meet the aerobic requirements. Further, an astounding 28% of Americans older than six are completely physically inactive.

One reason these goals are difficult to achieve for some Americans stems from occupations becoming decreasingly dependent on any sort of physical activity, while becoming increasingly demanding on longer working hours. A Gallup survey in 2014 demonstrated that all adults employed full-time in the U.S. work an average of 47 hours per week. These extra hours could have been utilized for a one-hour workout every day of the week, while still allowing time for daily chores and quality time with the family.

With time, sedentary practices coupled with the neglect of proper diet will expedite the rate at which Americans encounter cardiovascular diseases, such as stroke, congestive heart failure, coronary artery disease, and others. The latter is the most common type of heart disease, according to the Centers for Disease Control and Prevention, with close to 7% of Americans suffering from it in 2019. Coronary artery disease occurs when plaques (deposits of fat containing cholesterol) build up against the walls of major blood vessels, like the coronary arteries. This results in the narrowing of such arteries, which limit the blood flow to the heart and may lead to other complications, such as a heart attack.

Although this condition cannot be cured, prevention and treatment may help. Prevention includes following the aforementioned healthy lifestyle guidelines prior to developing the disease. Treatment includes addition of medication, angioplasty, and even invasive surgery along with the necessary lifestyle changes. With patients experiencing advanced coronary artery disease, surgical procedure is the best course of action to prevent a life-threatening scenario. A common procedure includes utilizing a portion of a healthy blood vessel from a different part of the patient's body as a new path for blood to flow around the blocked artery. This will allow the heart to receive a proper, unrestricted supply of blood. However, this is an invasive surgery which may lead to complications, and is therefore the ideal option when other, minimally invasive options have been ruled out.

Minimally invasive surgeries, according to Mayo Clinic, cause less overall damage to the body, involves lower risk of complications and infections, shorter recovery period, and provides the patient a level of comfort knowing the procedure is less threatening. Minimally invasive surgeries for treating CAD include the insertion of a balloon that widens the artery and allows more blood to flow through, or the use of a stent that serves a similar purpose to a balloon and prevents the artery from collapsing back to its original width. The balloon and stent are often placed together for maximum effectiveness. One shortcoming of this procedure is that it requires an incision to be made at the site of the stent insertion so that it may be properly placed in the blocked artery. This incision leaves the patient susceptible to infections and other complications.

Accordingly, there is an established need for a catheter design to permit trans-femoral access for carotid artery stent placement without any surgical incisions or general anesthesia, and which minimizes the risk of surgical site infection, avoids the need for blood transfusions, and reduces other surgical complications typically encountered with conventional carotid artery surgical approaches.

### Summary of the Invention

The present invention is directed to a medical instrument designed to provide interventional procedures such as trans-femoral access for carotid artery stent placement. The medical instrument includes, in combination, a catheter device and an introducer-type dilator. The catheter device includes a catheter sheath including a generally tubular body defining a lumen and having a proximal end, a distal end, and a sidewall construction. The sidewall construction has a multi-layered, laminate arrangement including an inner layer, an outer layer, and an intermediate layer disposed between the inner layer and the outer layer. In one illustrative form, the outer layer is preferably constructed with Nylon material and covered with hydrophilic coating for ease of advancement. The middle or intermediate layer is preferably constructed with braided stainless steel coil material to allow for adequate strength, which will minimize catheter kinking in tortuous vessels. The inner layer is preferably constructed with Poly-Tetra-Fluoro-Ethylene (PTFE) material to create a smooth surface for ease of device advancement. The sidewall construction further includes a micro-tube passageway defined at a radial location of the catheter sheath body. The passageway is interposed between the inner layer and the outer layer of the sidewall construction and extends axially between an inlet and an outlet thereof. The medical instrument further includes a valve mounted to the catheter sheath at the proximal end thereof. The valve includes an input side, an output side, a first side port, and a second side port. The input side of the valve is disposed in communication with the output side, and the output side is disposed in communication with the catheter sheath lumen.

In this manner, during operation, the valve is arranged to receive the dilator at its input side and to permit the dilator to advance axially into the lumen of the catheter sheath, where it projects a sufficient amount from the distal end of the catheter sheath to occupy a lead position and facilitate traversal of the catheter-dilator unit through a vascular vessel during application of the medical instrument in a vascular intervention.

The first port of the valve is disposed in communication with the passageway inlet, and the second port is disposed in communication with the catheter sheath lumen. The medical instrument further includes an inflatable occlusion balloon carried by the catheter sheath at the distal end thereof. The balloon has an inlet disposed in communication with the passageway outlet. The micro-tube passageway incorporated into the sidewall of the catheter sheath body functions as a hydraulic or pneumatic channel configured to transport a sufficient flow of pressurized liquid or gas, respectively, to the balloon to inflate it.

For this purpose, the first port of the valve is connected to a valve-regulated section of piping or tubing that includes a free end having an inlet orifice configured to admit a pressurized substance such as a mixture of contrast (dye)/normal saline. The fluid can be admitted using any suitable device such as a manual inflation syringe that removably connects to the tubing. During the inflation process, fluid admitted into the tubing enters the first port of the valve and travels to the balloon via the micro-tube passageway embedded in the sidewall of the catheter sheath body. The second port of the valve is connected to another valve-regulated section of piping or tubing that includes a free end having an orifice. In one form, this tubing is connected at its free end to a removable filtration system that is configured to filter blood products. During use of the medical instrument, the lumen of the catheter sheath receives a backflow or retrograde blood that travels from the distal end to the proximal end of the catheter sheath lumen, where they flow through the second port of the valve into the connected tubing and eventually reach the attached filtration system.

Introducing a first embodiment of the invention, the present invention consists of a device for percutaneous carotid artery stent placement through trans-femoral access, comprising:
a catheter sheath including a generally tubular body defining a lumen and having a proximal end, a distal end, and a sidewall construction;
the sidewall construction of the catheter sheath body having a laminate arrangement including an inner layer, an outer layer, and an intermediate layer disposed between the inner layer and the outer layer;
a passageway defined at a radial location of the catheter sheath body, wherein the passageway is interposed between the inner layer and the outer layer of the sidewall construction and extends axially between an inlet and an outlet thereof;
a valve mounted to the catheter sheath at the proximal end thereof, the valve includes an input side, an output side, a first port, and a second port, wherein the input side is disposed in communication with the output side, the output side is disposed in communication with the catheter sheath lumen, the first port is disposed in communication with the passageway inlet, and the second port is disposed in communication with the catheter sheath lumen; and
an inflatable balloon carried by the catheter sheath at the distal end thereof, the balloon having an inlet disposed in communication with the passageway outlet.

In another aspect, the balloon catheter is configured to selectively anchor the catheter sheath to surrounding tissue of the carotid artery when the balloon catheter is inflated. In exemplary embodiment, the anchoring of the catheter sheath prevents unwanted movement of the catheter sheath as retrograde blood flow from the carotid artery flows through the lumen of the catheter sheath and out of the second port that may be connected to a second conveyance unit that includes a filtering or pump system.

In another aspect, the catheter sheath may comprise of a sufficient length so as to extend the distance between a trans-femoral vein access site and a carotid artery of the patient. In some exemplary embodiments, the sufficient length is about 90cm.

In a further aspect of the invention, there is provided a device for percutaneous carotid artery stent placement through trans-femoral access, comprising: a catheter sheath including a generally tubular body defining a lumen and having a proximal end, a distal end, and a sidewall construction, the sidewall construction of the catheter sheath body includes a laminate arrangement including an inner layer, an outer layer, and an intermediate layer disposed between the inner layer and the outer layer; a passageway defined at a radial location of the catheter sheath tubular body, the passageway is interposed between an inner layer and an outer layer of the sidewall construction and extends axially between an inlet and an outlet thereof; a valve mounted to the catheter sheath at the proximal end thereof, the valve including a first port and a second port; and an inflatable balloon carried by the catheter sheath at the distal end thereof, the balloon having an inlet disposed in communication with the passageway.

The catheter sheath may be of a sufficient length to extend a distance between a trans-femoral access point and a carotid artery.

The balloon catheter may be configured to selectively anchor the catheter sheath to surrounding tissue of the carotid artery when the balloon catheter is inflated, the anchoring of the catheter sheath prevents unwanted movement of the catheter sheath as retrograde blood flow from the carotid artery flows through the lumen of the catheter sheath and out of the second port that is connected to a second conveyance unit.

In yet a further aspect of the present invention, there is provided a device for percutaneous carotid artery stent placement through trans-femoral access, comprising: a catheter sheath including a generally tubular body defining a lumen and having a proximal end, a distal end, and a sidewall construction, the sidewall construction of the catheter sheath body includes a laminate arrangement including an inner layer, an outer layer, and an intermediate layer disposed between the inner layer and the outer layer; a passageway defined at a radial location of the catheter sheath tubular body, the passageway is interposed between an inner layer and an outer layer of the sidewall construction and extends axially between an inlet and an outlet thereof; a valve mounted to the catheter sheath at the proximal end thereof, the valve including a first port and a second port; a first conveyance unit in communication with the first port, the first conveyance unit attachable to an injector device for inflating the inflatable balloon; a second conveyance unit in communication with the second port of the valve, the second conveyance unit attachable to a filtering system or pump system; and a marker disposed on the catheter sheath utilized for positioning the catheter sheath in a desired location in the carotid artery; and an inflatable balloon carried by the catheter sheath at the distal end thereof, the balloon having an inlet disposed in communication with the passageway, wherein the catheter sheath is of a sufficient length to extend a distance between a trans-femoral vein access site and the carotid artery, and wherein the balloon catheter is configured to selectively anchor the catheter sheath to surrounding tissue of the carotid artery when the balloon catheter is inflated, the anchoring of the catheter sheath prevents unwanted movement of the catheter sheath as retrograde blood flow from the carotid artery flows through the lumen of the catheter sheath and out of the second port that is connected to a second conveyance unit.

These and other objects, features, and advantages of the present invention will become more readily apparent from the attached drawings and the detailed description of the preferred embodiments, which follow.

### Brief Description of the Drawings

The preferred embodiments of the invention will hereinafter be described in conjunction with the appended drawings provided to illustrate and not to limit the invention, where like designations denote like elements, and in which:

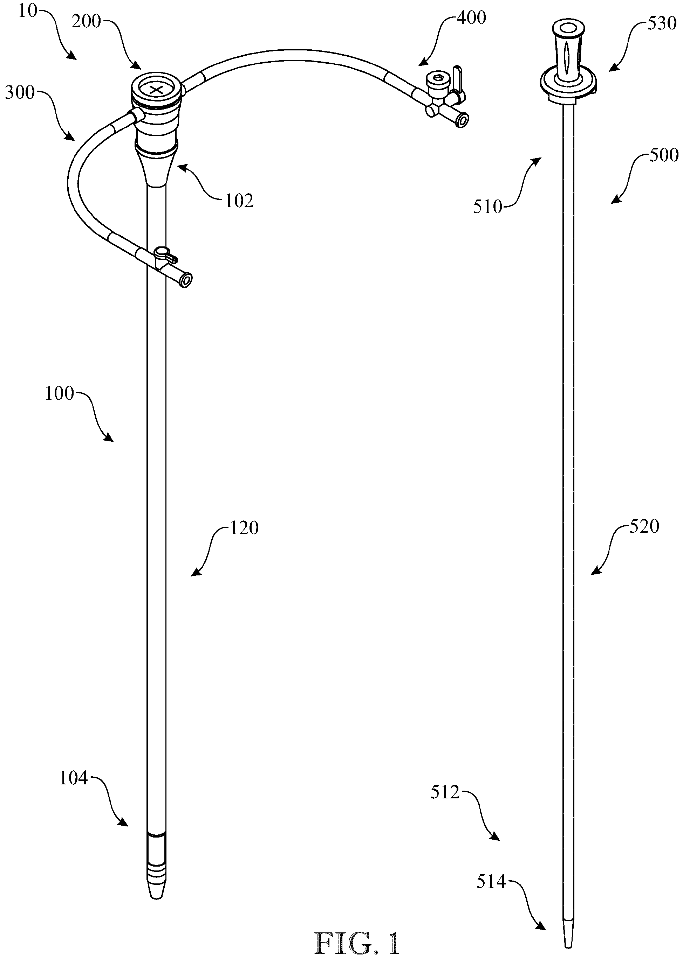

FIG. 1 presents a perspective view showing a first embodiment of the medical instrument of the present invention, showing the catheter assembly in combination with a dilator device that integrates with the catheter assembly during operation;

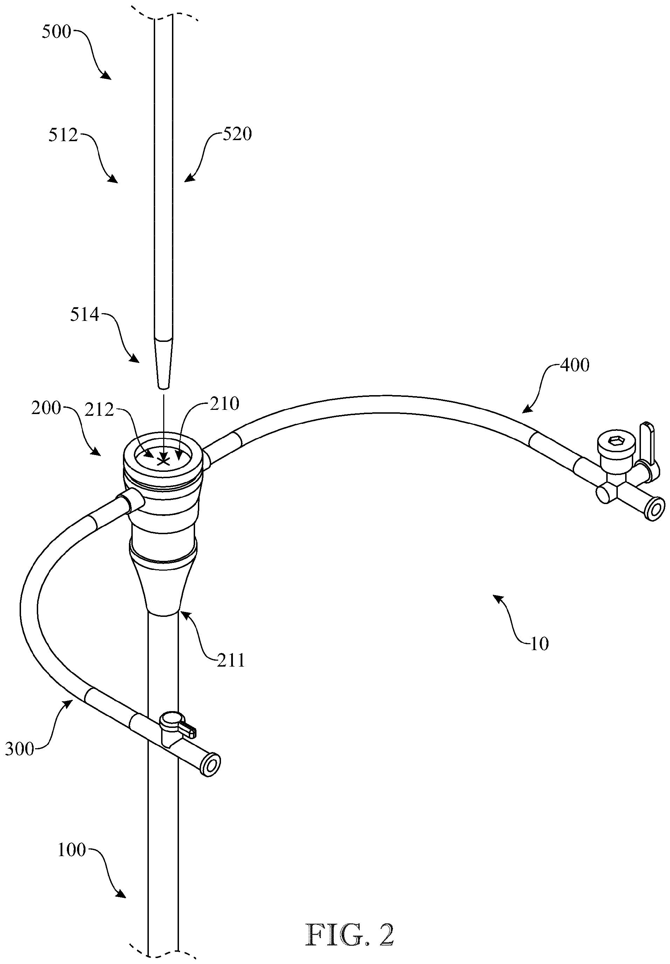

FIG. 2 presents a partial perspective view of the first embodiment of the medical instrument of the present invention originally disclosed in FIG. 1, showing the upper section of the catheter assembly and the alignment of the dilator device to the cross-cut valve of the catheter assembly prior to installation;

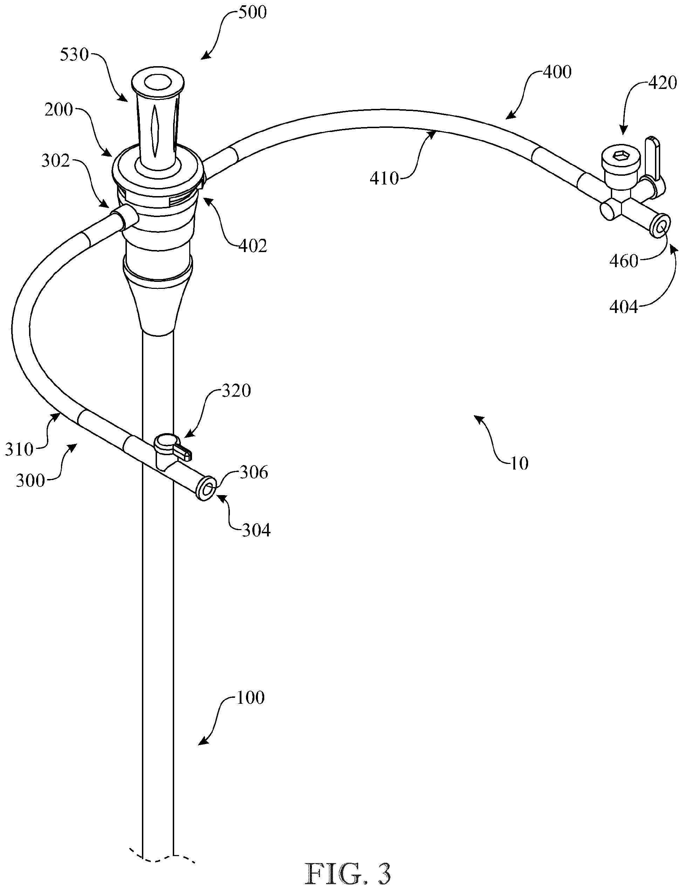

FIG. 3 presents a partial perspective view of the first embodiment of the medical instrument of the present invention originally disclosed in FIG. 1 and further depicted in FIG. 2, showing the completed installation of the dilator device in the catheter assembly via the cross-cut valve;

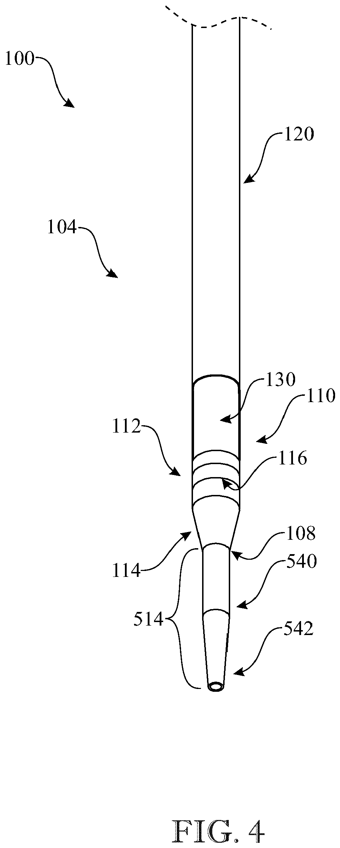

FIG. 4 presents a partial perspective view of the first embodiment of the medical instrument of the present invention originally disclosed in FIG. 1 and further depicted in FIG. 3, showing the lower end of the catheter assembly with an exposed portion of the dilator device extending axially from the end of the catheter sheath;

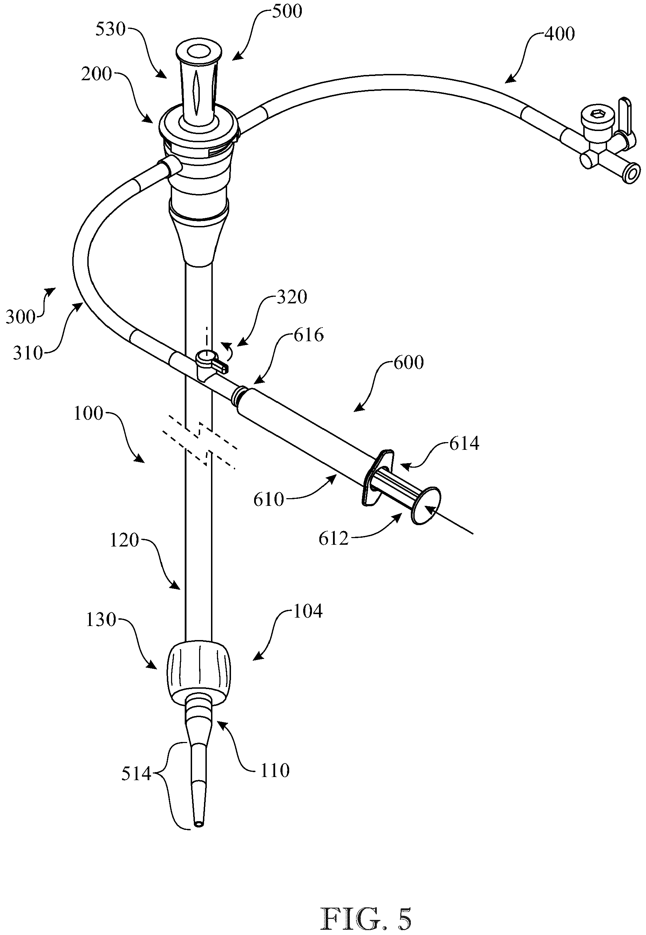

FIG. 5 presents a partial perspective view of the first embodiment of the medical instrument of the present invention originally disclosed in FIG. 1 and further depicted in FIG. 4, showing one operational mode in which an inflation device is connected to one of the ports of the cross-cut valve of the catheter assembly to facilitate inflation of an external occlusion balloon located at a lower end of the catheter sheath;

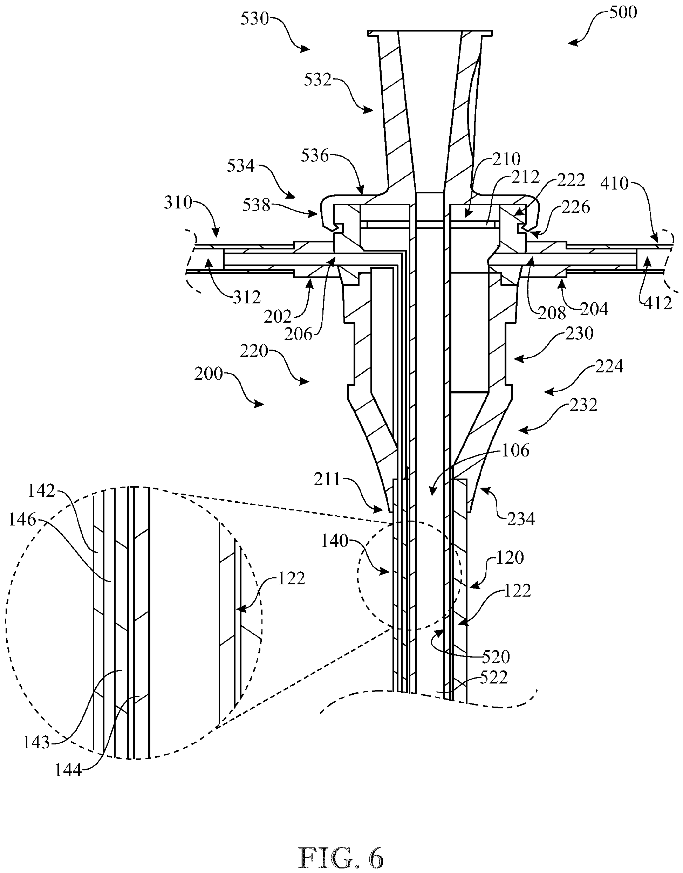

FIG. 6 presents a cross-sectional view of the cross-cut valve of the medical instrument according to the first embodiment of the present invention as originally disclosed in FIG. 1 and further depicted in FIG. 3, showing how one of the cross-cut valve ports is disposed in communication with a pneumatic passageway formed in the sidewall of the catheter sheath and further showing an enlarged sectional view of the catheter sheath sidewall;

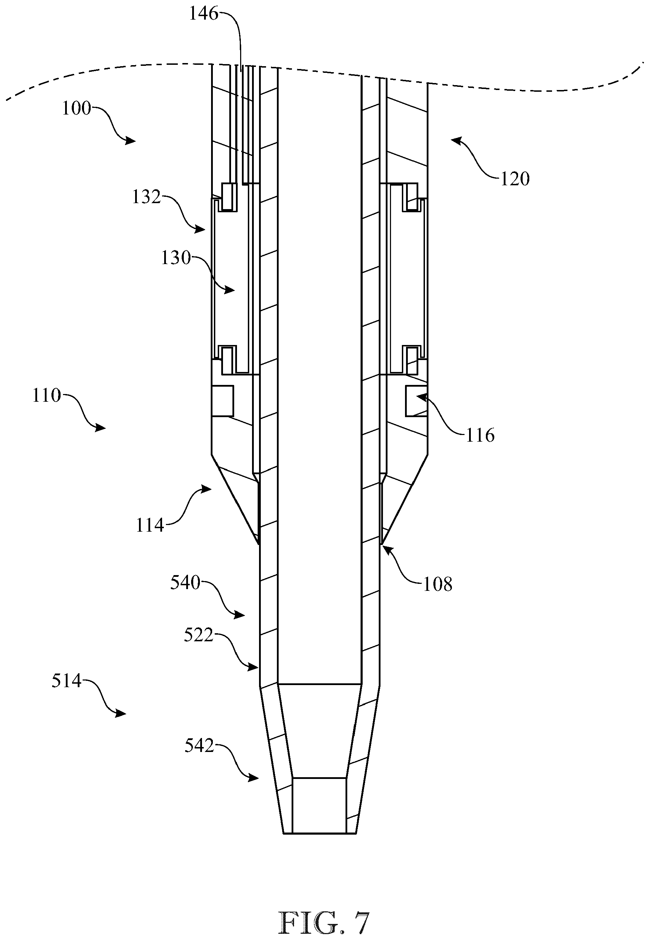

FIG. 7 presents a cross-sectional view of the distal end of the medical instrument according to the first embodiment of the present invention as originally disclosed in FIG. 1 and further depicted in FIGS. 3 and 4, showing the axial relationship of the catheter sheath and the dilator device following installation and further showing the location of the occlusion balloon in its deflated state; and

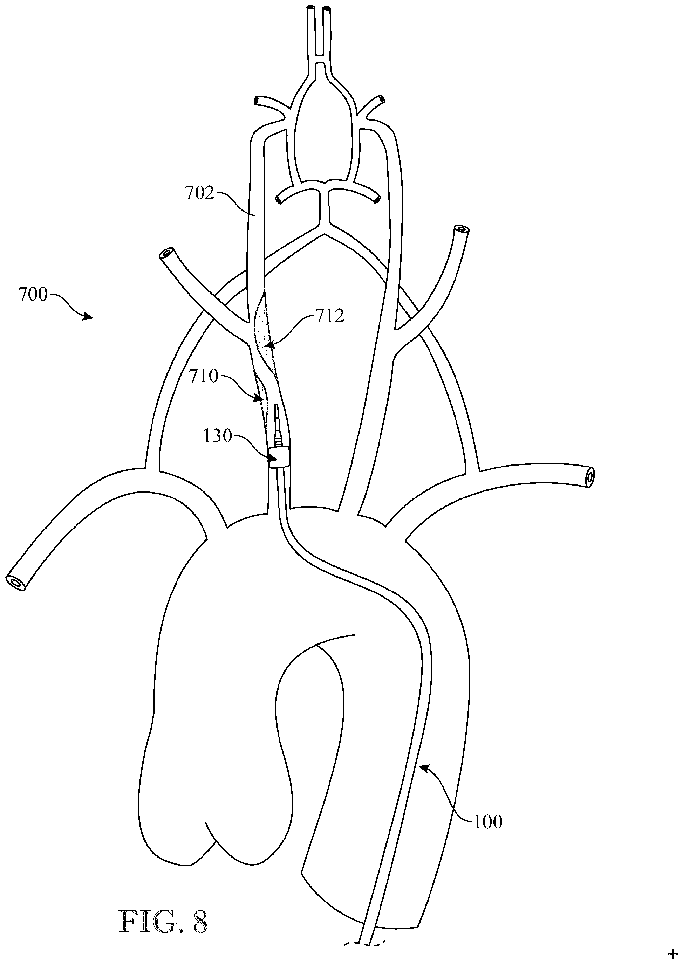

FIG. 8 presents a diagrammatic, anatomical, section view of a vascular system, showing an application of the medical instrument according to the first embodiment of the present invention originally disclosed in FIG. 1, in which the catheter assembly is disposed inside of a femoral artery then advanced superiorly to the common carotid artery and positioned inferiorly to a targeted lesion.

Like reference numerals refer to like parts throughout the several views of the drawings.

### Detailed Description

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper", "lower", "left", "rear", "right", "front", "vertical", "horizontal", and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Shown throughout the figures, the present invention is directed toward a vascular catheter assembly in combination with an introducer dilator device. The catheter assembly includes a catheter sheath having a sidewall construction formed as a multi-layered laminate arrangement. A dual-port cross-cut valve is mounted to the proximal end of the catheter sheath. A first valve port communicates with a micro-tube passageway that is formed in the sidewall construction and is adapted to access an occlusion balloon carried at a distal end of the catheter sheath. Fluid supplied through the first valve port is transported via the passageway to the balloon to inflate it. A second valve port communicates with the catheter sheath lumen, allowing blood products to backflow through the catheter sheath lumen and exit the valve to a filter system. The dilator accesses the catheter sheath lumen via the valve.

Referring initially to FIG. 1, a vascular medical instrument, device or appliance 10 is disclosed according to a first embodiment of the present invention. The instrument 10 includes, in combination, a catheter assembly 100, a cross-cut valve 200 disposed at a proximal end of catheter assembly 100, a first regulated conveyance unit 300 connected to one side port of valve 200, and a second regulated conveyance unit 400 connected to another side port of valve 200. The medical instrument 10 is used in combination with a removable, introducer-type dilator device 500. In particular, the dilator device 500 is part of an introducer set conventionally used to access blood vessels for the insertion of vascular catheters.

Briefly, in overview, the first conveyance unit 300 has a plumbing-style configuration that facilitates the passage of a hydraulic or pneumatic material into the catheter assembly 100 in order to inflate an occlusion balloon disposed at a distal end of catheter assembly 100. The second conveyance unit 400 has a plumbing-style configuration that facilitates the reception of body fluids (e.g., blood material) that enter the catheter assembly 100 from the anatomy of an individual under treatment and then routes the received blood material to an attachable filtering system (not shown). During use or application of medical instrument 10, the dilator device 500 is inserted telescopically into the lumen of the catheter assembly 100 via valve 200. In a conventional manner, during a vascular medical procedure or intervention, the combined catheter assembly 100 and dilator 500 are passed together into a blood vessel. The dilator device 500 is a removable apparatus that can be removed from its location within catheter assembly 100 once it has served its purpose. The medical instrument 10 is constructed with biocompatible materials to facilitate its use as an intervention tool with the human anatomy, particularly the vascular system.

Referring still to FIG. 1, in addition to FIGS. 4 and 6, the catheter assembly 100 includes a proximal end 102 where valve 200 is mounted and a distal end 104 that functions as the leading edge of catheter assembly 100 during procedural access. The catheter assembly 100 includes a catheter sheath or sleeve generally illustrated at 120 that defines the main body portion of catheter assembly 100 and extends between an upper opening or orifice 106 located at proximal end 102 and a lower opening or orifice 108 located at distal end 104. The sheath 120 has an elongate, generally tubular, hollow configuration that defines a longitudinal inner bore or lumen 122. The sheath 120 has a tubular, sidewall construction generally illustrated at 140 that incorporates a bio-compatible, multi-layered, laminate arrangement.

In one exemplary form, as shown in FIGS. 6, the sidewall construction 140 is formed with an axially-extending outer layer 142, inner layer 144, and an intermediate layer 143 interposed between the inner layer 144 and outer layer 142. In one illustrative form, the outer layer 142 is preferably constructed with a Nylon material and covered with hydrophilic coating for ease of advancement. In order to provide sheath reinforcement and stability, the middle or intermediate layer 143 is preferably constructed with braided stainless steel coil material to allow for adequate strength, which will minimize catheter kinking in tortuous vessels. The inner layer 144 is preferably constructed with Poly-Tetra-Fluoro-Ethylene (PTFE) material to create a smooth surface for ease of device advancement. In one form, the sheath 120 is approximately 90 cm long and the inner diameter of lumen 122 is approximate 0.114"/2.9mm (plus or minus 0.5 mm), although other sizes and dimensions are possible within the scope of the invention. The hydrophilic coating need not extend the full length of sheath 120 but may be applied, for example, to the distal 60cm of sheath 120. The sheath 120 preferably includes a gold-tipped, radio-opaque coil marker or marker band generally illustrated at 116 within transition portion 112, which may occupy a location approximately 2 mm from the terminus of sheath 120 and provide a conventional purpose in helping an operator sight the navigation of catheter assembly 100. The marker is highly visible with imaging equipment, such as a fluoroscope.

Referring to FIGS. 4 and 7, the distal end 104 of catheter sheath 120 includes a tip section generally illustrated at 110 that includes a transition portion 112 axially adjacent to a tapered terminus portion 114 that incorporates lower opening 108. The tip section 110 defines a free end of sheath 120. In one form, the tapered terminus portion 114 has a frusto-conical shape suitable to promote safe and easy advancement of catheter assembly 100 through vascular cavities and passageways (e.g., arteries and veins). The catheter assembly 100 is preferably equipped with an inflatable, external occlusion balloon generally illustrated at 130. In one form, the balloon 130 is carried by sheath 120 at an axial position approximately 5 mm distal from the sheath terminus. The balloon 130 is stored and otherwise loaded onto sheath 120 in its deflated condition with a surrounding, peripheral opening generally illustrated at 132 to permit expansion of balloon 130 during inflation. The balloon 130 has a design allowing it to be inflated to different sizes at a low atmospheric pressure, which permits it to conform atraumatically to the size of the selected vessel. The balloon 130, in one form, can be inflated to accommodate vessel sizes between 5-12 mm. The balloon 130 is preferably constructed utilizing compliant material allowing for even distribution of the pressurizing saline/contrast mixture.

Referring to FIGS. 1-3 and 6, the catheter assembly 100 includes a cross-cut valve 200 preferably having a conventional construction. The valve 200 includes a generally cylindrical body generally illustrated at 220 having an upper side 210 and a lower side 211. The valve upper side or end 210 defines an entry way or opening to receive introducer dilator device 500. The valve lower side or end 211 defines an exit opening through which the inserted dilator 500 can advance into the lumen 122 of catheter sheath 120. The valve 200 includes a sealable entrance membrane generally illustrated at 212 that spans the entrance opening in valve upper side 210. The valve body 220 includes an upper section generally illustrated at 222 and a contiguous lower section generally illustrated at 224. The valve upper section 222 defines a mounting head or platform to receive dilator 500 and establish a releasable attachment to dilator 500 using a quick-release, snap-fit locking engagement. The valve lower section 224 is adapted to receive, secure, and otherwise hold catheter sheath 120 at its proximal end 102. The valve lower section 224 includes a generally cylindrical upper portion generally illustrated at 230 and a generally tapered or reduced diameter lower portion generally illustrated at 232. The terminal part of lower tapered portion 232 is configured with an enlarged diameter to form a longitudinally-extending slot having an axial abutment in the form of a shoulder structure. This enlarged diameter section defines a sheath-receiving and holding collar generally illustrated at 234, which receives sheath 120 at its proximal end 102 in a telescoping, concentric arrangement or relationship.

Referring more particularly to FIG. 6, with continuing reference to FIGS. 1 and 3, the valve 200 further includes a first side port generally illustrated at 202 and a second side port generally illustrated at 204. The first side port 202 and second side port 204 are adapted for external connection to a first regulated conveyance unit 300 and a second regulated conveyance unit 400, respectively. The second side port 204 is configured with a channel 208 that communicates at one end with lumen 122 of catheter sheath 120 and communicates at another end with second regulated conveyance unit 400. The second conveyance unit 400 includes a tubing or piping 410 that extends between a free, output end 404 having an opening or orifice 406 and a valve-attached, input end or opening 402 disposed in communication with second side port 204 of valve 200 via the hollow interior space 412 of tubing 410. The second conveyance unit 400 is removably attached to valve 200 at second side port 204. The second conveyance unit 400 is removably coupled at its free end 404 to a filtration system (not shown).

During operation, any blood products that enter lumen 122 of catheter sheath 120 can migrate in a reverse flow from distal end 104 to proximal end 102, where the flow of blood products can exit valve 200 via second side port 204, route through tubing 410, and enter the attached filtration system (or pump unit system). The second conveyance unit 400, in one exemplary embodiment, includes an inline valve regulator 420 to regulate the retrograde blood flow traveling through tubing 410. In one form, the side ports 202, 204 can be designed with an 8F (French) size, 0.114"/2.9 mm inner lumen diameters, compatible with a similarly sized and dimensioned tubing 410. The variable-flow valve regulator 420 may have any suitable configuration including, for example, a 3-way stop-cock design featuring an open position (100% flow), partially open/partially closed (e.g., 50% flow), and closed (no flow). Other variable and adjustable flow rates are possible. The second conveyance unit 400 effectively functions as a docking system to permit the fluid connection of external appliances (such as a filtration system) to the catheter sheath 120 via the intermediate coupling of cross-cut valve 200.

Referring to FIGS. 6 and 7, the catheter assembly 100 is further adapted to provide a fluid line that supplies balloon 130 with hydraulic and/or pneumatic pressurization to effectuate controllable and adjustable inflation/deflation of balloon 130. For this purpose, in one form, the sidewall construction 140 of catheter sheath 120 incorporates a passageway 146 formed between inner layer 144 and outer layer 142 of the multi-layered laminate arrangement of catheter sheath 120. The passageway 146 is disposed at a distal end in fluid communication with balloon 130 (FIG. 7) and disposed at a proximal end in fluid communication with first side port 202 of cross-cut valve 200 (FIG. 6). The first side port 202 is configured with a channel 206 that communicates at one end with the balloon-pressurizing passageway 146 of catheter sheath 120 and communicates at another end with first regulated conveyance unit 300. The first conveyance unit 300 includes a tubing or piping 310 that extends between a free, input end 304 having an opening or orifice 306 and a valve-attached, output end or opening 302 disposed in communication with first side port 202 of valve 200 via the hollow interior space 312 of tubing 310. The first conveyance unit 300 is removably attached to valve 200 at first side port 202. The first conveyance unit 300 is removably coupled at its free end 304 to a syringe-type injector device generally illustrated at 600, which supplies a pressurized, adjustable flow of pneumatic and/or hydraulic fluid.

Referring to FIG. 5, the injector device 600 can have any conventional construction. In one form, the injector device 600 includes, in combination, an elongate barrel or cylindrical tube 610 and a plunger or piston 612 that fits telescopically into barrel 610 via entry opening 614. The barrel 610 has a discharge orifice or open front end generally illustrated at 616, which is configured in releasable fluid communication with pipe 310 of first conveyance unit 300. The barrel 610 is filled with a suitable fluid, such as a mixture of contrast (dye)/normal saline. During operation of injector device 600, pressurized fluid is admitted into tubing 310 of first conveyance unit 300, enters valve 200 via first side port 202, and is transported via the sheath-embedded micro-tube passageway 146 to balloon 130, causing balloon 130 to inflate. The first conveyance unit 300 preferably includes an inline valve regulator 320 to regulate the fluid flow traveling through tubing 310 and hence adjust the pressurization of balloon 130.

Referring now to FIGS. 1-4 and 6-7, the catheter assembly 100 of medical instrument 10 is used in combination with an introducer-type dilator device 500 to form an integral set. The dilator 500 can have any suitable conventional construction. In one form, dilator 500 includes an elongate, tubular main body portion or shaft 520 defining a lumen 522 and having a proximal catheter-attaching end 510 and a distal catheter-guiding end 512. The dilator 500 further includes a combined handle and attachment head generally illustrated at 530 and disposed at the proximal end 510. Referring to FIG. 6, the head section 530 includes a handle portion 532 and an attachment portion 534 configured to facilitate releasable mounting of dilator 500 to valve 200. The attachment portion 534 includes a generally planar, disc-shaped body 536 joined to handle portion 532 and an inwardly-curving peripheral lip 538 extending axially from disc-shaped body 536 and then extending radially towards and into (during attachment) a peripheral, dilator-receiving and catching recess 226 formed in the upper section 222 of valve body 220. Preliminary to the interventional procedure, dilator 500 is inserted into catheter assembly 100 to form a single integrated unit that functions as a vascular introducer set designed to access blood vessels and facilitate the insertion and navigation of the catheter sheath 120. To facilitate vascular access and advancement, dilator 500 includes a terminal tip section 514 at distal end 512. The tip section 514 includes an upper portion 540 contiguous with a lower tapered terminus portion 542, which serves as the leading edge of dilator 500 for insertion and access purposes.

During assembled installation, dilator 500 is inserted into cross-cut valve 200 by maneuvering tip section 514 through the seal-type membrane 212 located in the upper side 210 of valve 200. For this purpose, membrane 212 is equipped with a flexible, resilient flap configuration that is breached by the advancing tip section 514 to permit dilator 500 to advance into valve 200, and then closes around the shaft 520 of dilator 500 in sealable fashion (FIG. 2). The cross-cut valve 200 maintains a seal around any device being introduced. This feature will prevent back bleeding or air entry into the flow reversal sheath 120 during the intervention procedure.

Once inserted into valve 200, dilator 500 continues its advance until the mounting head 530 of dilator 500 becomes secured and seated in a releasable, snap-on, locking engagement with the upper section 222 of valve body 220 (FIGS. 3 and 6). This locking attachment between dilator 500 and cross-cut valve 200 maintains dilator 500 in place to prevent back-out from happening during use. As dilator 500 advances through catheter assembly 100 via cross-cut valve 200 during installation, the shaft 520 of dilator 500 travels through the lumen 122 of catheter sheath 120 (FIG. 6). In its installed condition, the dilator 500 extends axially beyond the terminus of catheter sheath 120. In particular, the tip section 514 of dilator 500 projects from the lumen 122 of catheter sheath 120 (FIGS. 4, 5, 7). In this manner, the tip section 514 of dilator 500 functions as the leading edge of the combined unit. Once it has served its guiding function, the dilator 500 can be withdrawn from its telescoped location within catheter assembly 100. The design of cross-cut valve 200 permits other devices to be inserted in and through lumen 122 of catheter sheath 120, such as wires, stents, inflation balloons, and additional catheter appliances.

The dilator 500, and particularly the tapering profile of tip section 514, facilitates the introduction and safe positioning of catheter sheath 120. The dilator 500 is concentrically located within lumen 122 of catheter sheath 120, advancing through the cross-cut valve 200, traversing the entire length of catheter sheath 120, and protruding out of the distal end 104 of catheter sheath 120 (by approximately 2 cm, for example). The inner-located dilator 500 is preferably made of kink-resistant polypropylene material or any other suitable construction well known to those skilled in the art. The lumen 522 of dilator 500 extends the entire length of dilator shaft 520, open at both its proximal and distal ends 510, 520. The dilator lumen 522 is designed to accommodate guide wires of varying sizes. The dilator 500 fits seamlessly and precisely through the main flow reversal catheter sheath 120, not allowing the accumulation of blood or air particles to be entrapped between dilator 500 and the inner diameter surface of catheter sheath 120. In a conventional manner, the catheter sheath 120 and dilator 500 are introduced as a single unit over an indwelling guide wire positioned within the lumen 522 of dilator 500. The dilator 500 is utilized during the initial flow reversal sheath advancement, which is preferably performed under fluoroscopic guidance.

FIG. 8 depicts one illustrative operation for locating catheter assembly 100 in relationship to exemplary lesion sites 710, 712 present in the carotid artery 702 of an anatomical vascular system 700. Further procedural interventions can take place once catheter assembly 100 is positioned in proximity to lesion sites 710, 712, such as stent placement. As illustrated in the referenced figures and described hereinabove, the catheter is designed and otherwise configured to have a sufficient length to permit trans-femoral access for carotid artery stent placement without any surgical incisions or general anesthesia. Sufficient length is defined as the distance extending between a trans-femoral access point and the affected carotid artery. The catheter's design minimizes the risk of surgical site infection, avoids the need for blood transfusions, and reduces other surgical complications typically encountered with conventional carotid artery surgical approaches, as known by those of ordinary skill in the art.

Since many modifications, variations, and changes in detail can be made to the described preferred embodiments of the invention, it is intended that all matters in the foregoing description and shown in the accompanying drawings be interpreted as illustrative and not in a limiting sense. Furthermore, it is understood that any of the features presented in the embodiments may be integrated into any of the other embodiments unless explicitly stated otherwise. The scope of the invention should be determined by the appended claims and their legal equivalents.

## Claims

1. A device for percutaneous carotid artery stent placement through trans-femoral access, comprising:
a catheter sheath including a generally tubular body defining a lumen and having a proximal end, a distal end, and a sidewall construction;
a passageway defined at a radial location of the catheter sheath body extending throughout the tubular body;
a valve mounted to the catheter sheath at the proximal end thereof, the valve including a first port and a second port; and
an inflatable balloon carried by the catheter sheath at the distal end thereof, the balloon having an inlet disposed in communication with the passageway.

2. The device of claim 1, wherein the sidewall construction of the catheter sheath body includes a laminate arrangement including an inner layer, an outer layer, and an intermediate layer disposed between the inner layer and the outer layer.

3. The device of claim 1 or 2, wherein the passageway is interposed between an inner layer and an outer layer of the sidewall construction and extends axially between an inlet and an outlet thereof.

4. The device of any preceding claim, wherein the first port communicates with the passageway of the catheter sheath.

5. The device of any preceding claim, wherein the second port communicates with the catheter sheath lumen.

6. The device of claim 4, wherein a first conveyance unit is disposed in communication with the first port, the first conveyance unit is attachable to an injector device for inflating the inflatable balloon.

7. The device of any preceding claim, wherein the balloon catheter is inflatable

8. The device of claim 5, wherein a second conveyance unit is disposed in communication with the second port, the second conveyance unit attachable to a filtering system or pump system.

9. The device of any preceding claim, wherein the catheter sheath is of a sufficient length to extend a distance between a trans-femoral vein access site and a carotid artery.

10. The device of claim 9, wherein the sufficient length is about 90cm.

11. The device of any preceding claim, wherein the catheter sheath includes a marker utilized for positioning the catheter sheath in a carotid artery.

12. The device of claim 11, wherein the marker is a gold-tipped, radio-opaque coil marker.

13. The device of claim 11 or 12, wherein the marker is positioned on the catheter sheath at an inferior placement to the distal end of the catheter sheath but at a superior placement to the balloon catheter.

14. The device of any preceding claim, wherein the balloon catheter is configured to selectively anchor the catheter sheath to surrounding tissue of the carotid artery when the balloon catheter is inflated, the anchoring of the catheter sheath prevents unwanted movement of the catheter sheath as retrograde blood flow from the carotid artery flows through the lumen of the catheter sheath and out of the second port that is connected to a second conveyance unit.

15. The device of claim 14, wherein the conveyance unit comprises at least one tubing, a control valve connected to an end of the tubing, and a filtering system connected to the control valve.

16. The device of claim 14 or 15, wherein a stent placement device is insertable into the lumen of the catheter sheath through the valve for positioning a stent in the carotid artery during retrograde blood flow.
